# EUROPEAN PATENT APPLICATION

(11) **EP 2 147 694 A1**
(43) Date of publication of application: **27.01.2010**
(21) Application number: 09166293.2
(22) Date of filing: 24.07.2009
(51) Int. Cl.: A61M 25/092, A61M 25/00

(54) **Steerable catheter and method of making the same**

(30) Priority: 25.07.2008 US 220621
(71) Applicant: NeuroTherm, Wilmington, MA 01887 (US)
(72) Inventor: Parasmo, Ronald, North Palm Beach, FL 33410 (US); Renick, Michael A., Lake Park, FL 33403 (US)
(74) Representative: Reichert, Werner Franz

(57) **Abstract**

A steerable catheter (100) including a helical coil (120) of wire (126) having a longitudinal axis (A-A), a distal end (129), a proximal end (121), and a lumen (125) extending longitudinally from the distal (129) end to the proximal end (121), a ribbon (140) having a distal end (129), and extending longitudinally, adjacent to the longitudinal axis (A-A), within the lumen (125), from the distal end (129) of the helical coil (120) to the proximal end (121) of the helical coil (120), and a tip formed by a laser-welded fusion of the distal end (129) of the helical coil (120) and the distal end (129) of the ribbon (140), the tip being arranged to close the distal end (129) of the helical coil (120), and operatively arranged to move in a first direction away from the longitudinal axis (A-A) when the ribbon (140) is forced in a first longitudinal direction, and move in a second direction away from the longitudinal axis (A-A) when the ribbon is forced in a second longitudinal direction.

## Description

The invention broadly relates to catheters. More particularly, the invention relates to coil catheters and, even more particularly to steerable coil catheters.

Coil catheters are well known and commonly used, particularly for epidural injections. Generally, a coil catheter comprises a stainless steel, helical coil having a lumen, and a plurality of openings near its distal end for delivering drugs into the surrounding space and tissues. Commonly, its distal end is closed via a tip assembly comprising an initially separate tip that is fixed to the distal end of the coil via some type of adhesive or welding.

One problem with prior coil catheters is that the tips have a tendency to detach from the coil while the catheter is still inserted within a patient. This can lead to whole host of medical problems, as well as product liability and medical malpractice lawsuits. Often, a tip detaches because the means for affixing it to the end of the coil is inadequate, due to the general inability to assemble and connect such small components. This problem is exacerbated when the attached tip has a diameter greater than the diameter of the coil, as the oversized tip tends to get caught on tissue, which can lead to tissue damage during operation, and on the end of the introducers used to provide the catheter access into the target tissue and epidural space.

Another problem with prior coil catheters is that they are difficult to direct after they have been inserted into the target area. Generally, a stylet comprising a wire is inserted into the lumen of the coil at the proximal end, and is used to provide some rigidity to the flexible coil and direct the tip through the tissue to the target area. A stylet is only operatively arranged to direct the tip linearly forward, and provides no means for redirecting the tip in a different direction. This drawback prevents a user from steering the catheter in and around structures, such as, spinal nerves and bone processes, which limits his ability to effectively reach target tissues he wishes to expose to drugs, such as, anti-inflammatories, steroids, analgesics, *etc*.

Thus, there is a long-felt need for a coil catheter having a non-detachable tip. There is also a long-felt need for a steerable coil catheter.

The present invention broadly is a steerable catheter comprising: a helical coil of wire, the helical coil comprising an outer diameter; an inner diameter; a longitudinal axis; a distal end; a proximal end; and, a lumen extending longitudinally from the distal end to the proximal end, the helical coil having at least a first section of loosely coiled loops defining at least one gap; a ribbon having a distal end and a proximal end, the ribbon extending longitudinally, adjacent to the longitudinal axis, within the lumen, from the distal end of the helical coil to the proximal end of the helical coil; a tip comprising a laser-welded fusion of the distal end of the helical coil and the distal end of the ribbon, the tip being arranged to close the distal end of the helical coil, and having a diameter substantially equal to or less than the outer diameter of the helical coil, wherein the tip is operatively arranged to move in a first direction away from the longitudinal axis when the ribbon is forced in a first longitudinal direction, and move in a second direction away from the longitudinal axis when the ribbon is forced in a second longitudinal direction.

The present invention also includes a method of making a steerable catheter tip comprising: providing a helical coil of wire of laser weldable material, the helical coil comprising a longitudinal axis, a distal end, a proximal end, and a lumen extending longitudinally from the distal end to the proximal end; providing a ribbon of laser weldable material having a distal end and a proximal end, the ribbon extending longitudinally, adjacent to the longitudinal axis, within the lumen, from the distal end of the helical coil past the proximal end of the helical coil; applying a first set of pulses from a laser welder to the distal end of the ribbon and the distal end of the helical coil, the first set of pulses being sufficient to generate a molten fusion of the distal end of the ribbon and the distal end of the helical coil; and, applying a second set of pulses from a laser welder to the molten fusion sufficient to shape the molten fusion into a tip arranged to close the distal end of the helical coil.

It is a general object of the present invention to provide a coil catheter having a non-detachable tip.

It is another general object of the present invention to provide a steerable coil catheter.

These and other objects and advantages of the present invention will be readily appreciable from the following description of preferred embodiments of the invention and from the accompanying drawings and claims.

The nature and mode of operation of the present invention will now be more fully described in the following detailed description of the invention taken with the accompanying drawing figures, in which:
Figure 1 is a perspective view of the present invention steerable catheter;
Figure 2 is a side plan view of a helical coil and a control ribbon of the steerable catheter, showing the first and second steps in the method of making the steerable catheter;
Figure 3 is a side plan view of the coil and control ribbon, showing the third step in the method of making the steerable catheter;
Figure 4 is a side plan view of the coil and control ribbon, showing the fourth step in the method of making the steerable catheter;
Figure 5 is a side plan view of the steerable catheter in resting state;
Figure 6 is a side plan view of the steerable catheter, showing its tip steered in a first direction; and,
Figure 7 is a side plan view of the steerable catheter, showing its tip steered in a second direction.

At the outset, it should be appreciated that like drawing numbers on different drawing views identify identical, or functionally similar, structural elements of the invention. While the present invention is described with respect to what is presently considered to be the preferred aspects, it is to be understood that the invention as claimed is not limited to the disclosed aspects.

Furthermore, it is understood that this invention is not limited to the particular methodology, materials and modifications described and as such may, of course, vary. It is also understood that the terminology used herein is for the purpose of describing particular aspects only, and is not intended to limit the scope of the present invention, which is limited only by the appended claims.

Unless defined otherwise, all technical and scientific terms used herein have the same meaning as commonly understood to one of ordinary skill in the art to which this invention belongs. Although any methods, devices or materials similar or equivalent to those described herein can be used in the practice or testing of the invention, the preferred methods, devices, and materials are now described.

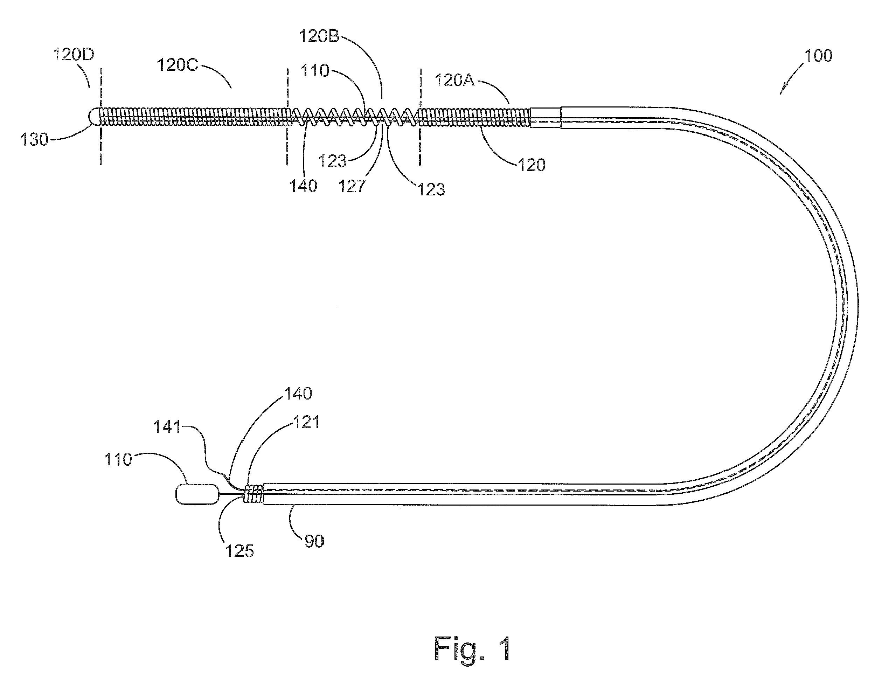

The following description of is best understood in view of Figures 1-7. Figure 1 shows steerable catheter **100** broadly comprising helical coil **120,** control ribbon **140,** and tip **130.** In preferred embodiments, steerable catheter **100** further comprises coating **90,** which covers a portion of helical coil **120,** and stylet **110.** Stylet **110** preferably comprises a stainless steel wire and molded plastic hub, wherein the wire is insertable in one end of helical coil, and is arranged to provide the rigidity necessary to push catheter **100** through tissue.

Helical coil **120** comprises wire **126** helically arranged in series of loops **123,** which are preferably substantially uniform. Helical coil **120** includes an outer diameter, an inner diameter, longitudinal axis **A-A',** distal end **129,** proximal end **121,** and lumen **125,** which extends longitudinally from distal end **129** to proximal end **121.** Helical coil **120** preferably includes proximal section **120A,** in which loops **123** are tightly coiled, at least a first section **120B** of loosely coiled loops **123,** distal section **120C,** which, prior to the formation of tip **130,** as described in detail *infra*, includes terminal section **120D.** Loops **123** of section **120B** define at least one, but preferably eleven to thirteen (11-13), gaps **127** which are operatively arranged to allow fluids to pass between lumen **125** and the exterior of helical coil **120.** It is contemplated that helical coil **120** may comprise a plurality of loosely coiled sections, such as section **120B,** in order to provide multiple drug delivery sites.

Wire **126** may comprise any material suitable for laser welding, such as, steels, nickel alloys, titanium, some aluminum alloys, and copper, but preferably comprises medical grade 304V stainless steel. Further, wire **126** comprises a substantially circular cross-section having a diameter ranging from 0.001-0.005 inches (in.), as a substantially planar, ribbon-like wire may provide insufficient material and structure when forming tip **130,** as described in more detail *infra.* Depending on the intended catheter size, wire **126** must be the correct size within 0.0001 in. Wire **126** should be inspected for surface abrasions, as there cannot be any imperfections in the surface finish. The process of forming wire into a helical coil via an arbor having progressive feed controls, predetermined stacking parameters, spring collection means, and a support tube is well known and, therefore, not reiterated herein. Preferably, helical coil **120** has an outer diameter ranging from approximately 0.015-0.027 in., and an inner diameter ranging from 0.011-0.017 in.

Preferably, catheter **100** includes coating **90** covering at least a portion of section **120A,** with a thickness ranging from 0.0005-0.002 in. A coated section of helical coil **120** preferably has a total outer diameter ranging from 0.016-0.031 in. Coating **90** may be any polymeric, heat-shrink tubing suitable for use on a catheter.

Control ribbon **140** is preferably a ribbon comprising any material suitable for laser welding, as described *supra,* but preferably medical grade 304V stainless steel. Control ribbon preferably has a width ranging from 0.002-0.005 in., and comprises distal end **149** and proximal end **141,** and extends longitudinally, adjacent to longitudinal axis **A-A',** within lumen **125,** from distal end **129** to proximal end **121** of helical coil **120.** In a preferred embodiment, control ribbon **140** extends past proximal end **121.**

Tip **130** comprises laser-welded fusion **131** of distal end **129** of helical coil **120** and distal end **149** of control ribbon **140,** tip **140** being arranged to close distal end **129.** Preferably, tip **130** is substantially spherical, but may also be hemi-spherical, conical, frustoconical, etc. Further, tip **130** has a diameter substantially equal to or less than the outer diameter of helical coil **120.** The method of making tip **140,** which is considered an aspect of the present invention, is described in more detail *infra.*

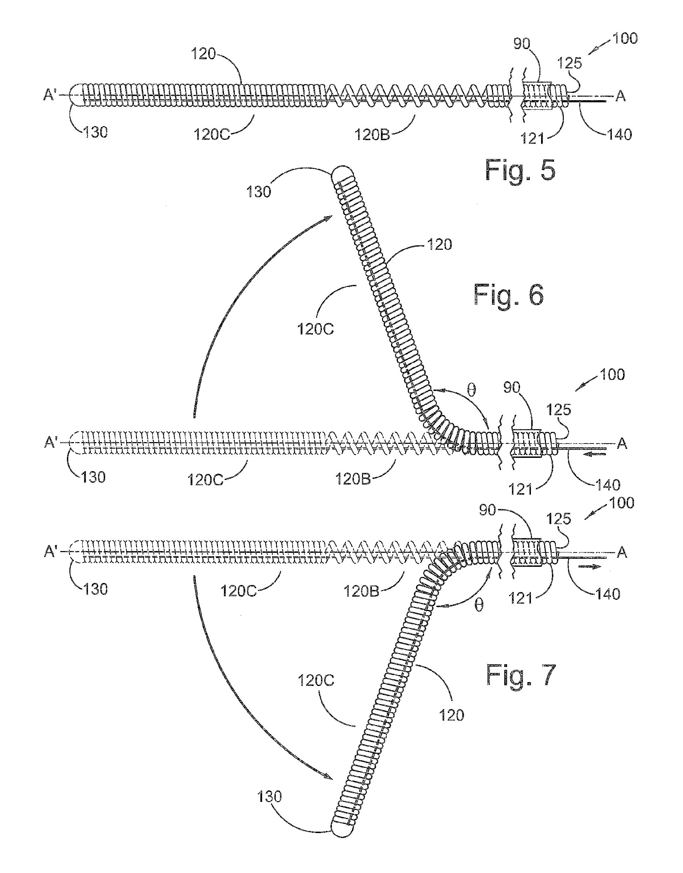

The steerability of catheter **100** is provided, in part, be the off-center arrangement of control ribbon **140** within lumen **125.** Since control ribbon **140** is arranged longitudinally and adjacent to longitudinal axis **A-A',** it extends from tip **130** at an off-center position. With this arrangement, tip **130** is operatively arranged to move and/or deflect in a first direction away from longitudinal axis **A-A'** when control ribbon **140** is forced in a first longitudinal direction, for example, when it is forced in a direction toward tip **140,** as illustrated in Figure 6. Additionally, tip **130** is operatively arranged to move and/or deflect in a second direction away from longitudinal axis **A-A'** when control ribbon **140** is forced in a second longitudinal direction, for example, when it is forced in a direction away from tip **130,** as illustrated in Figure 7. The movement and/or deflection of tip **130** away from axis **A-A'** is also provided by the flexibility of helical coil **120,** particularly at section **120B**, wherein gaps **127** allow adjacent loops **123** to shift independently from each other. In the embodiment shown in Figures 5-7, helical coil **120** is arranged to bend at angle θ, which is less than or equal approximately seventy degrees (70°), when control ribbon **140** is forced in the first and/or second longitudinal direction.

Steerable catheter **100** is formed utilizing via the following hereinbelow. The method involves laser welding, preferably with a pulse Nd-YAG (neodymium-yttrium aluminum garnet) laser welder running at 33.5 watts (W). As will be readily apparent to those have skill in the relevant art, the particular power ramping, pulse repetition, shaping, and width, and laser parameters used in the method will primarily depend upon the particular materials and dimensions selected for helical coil **120** and control ribbon **140.**

Regardless of the particular materials and/or dimensions selected, the method of making steerable catheter **100** comprises the following steps.

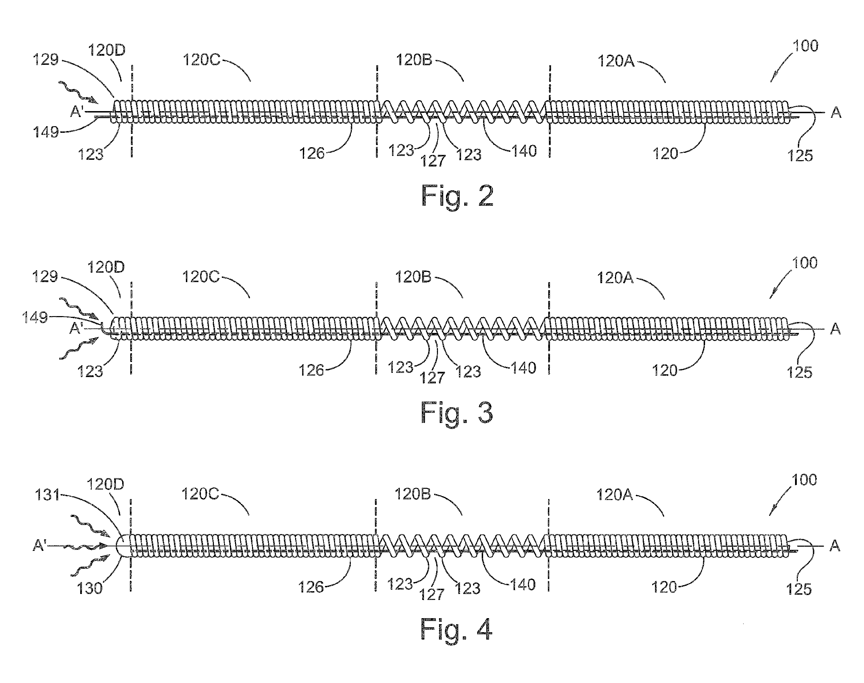

First, helical coil **120** is provided and held stationary, for example, using a fixture clamp (not shown). Second, control ribbon **140** is provided and arranged longitudinally within lumen **125** such that distal end **149** is proximate distal end **129.** The first two steps are illustrated in Figure 2.

Third, as shown in Figure 3, the laser of the laser welder is focused on distal end **149,** as depicted with the curved arrows, and applies a first set of pulses sufficient to weld distal end **149** to distal end **129.** It should be readily apparent to those having skill in the art that an inert shielding gas, such as argon, should be applied to the target area of the laser in order to protect the weld from the oxygen and nitrogen present in the air.

As stated *supra*, the particular pulse repetition rate and pulse widths applied during this step will depend on the selected materials and dimensions of helical coil **120** and control ribbon **140.** However, an important purpose of this step is to weld distal end **149** of control ribbon **140** at the previously describe off-center position, which is best accomplished by selecting a pulse repetition rate and pulse width that is sufficient to weld distal end **149** to distal end **129,** while minimizing the conversion of distal end **129** to a molten state. This aspect is illustrated in Figure xx which shows distal end **149** welded to one side of distal end **129,** with minimal deformation of distal end **129.** This figure also shows distal end **149** curled over lumen **125** as begins to become molten. In this way, control ribbon **140** also serves as a filler rod providing the initial material needed to bridge the gap between the loops **123** of terminal section **120D.**

Fourth, as shown in Figure 4, the laser applies a second set of pulses, as depicted with the curved arrows, to the curled distal end **149** and the loops **123** of terminal section **120D.** The second set of pulses should be sufficient to form a molten fusion of distal end **149** and distal end **129,** including terminal section **120D.** The number of coils **123** should be selected to provide enough molten material, when combined with the molten distal end **149** of control ribbon **140,** to shape the fusion into tip **130,** such that tip **130** is arranged to close distal end **129.** The second set of pulses should also be selected to form tip **140** into a uniform shape, such as a sphere, hemi-sphere, cone, frustum, *etc*., having a diameter substantially equal to or less than the outer diameter of helical coil **120.**

Tip **130** should be formed such that it is operatively arranged to move in a first direction away from longitudinal axis **A-A'** when control ribbon **140** is forced in a first longitudinal direction, and to move in a second direction away from longitudinal axis **A-A'** when control ribbon **140** is forced in a second longitudinal direction. Preferably, control ribbon **140** should be pull tested after tip **130** to at least 0.2 pounds per square inch (lbs/in.²).

Thus, it is seen that the objects of the present invention are efficiently obtained, although modifications and changes to the invention should be readily apparent to those having ordinary skill in the art, which modifications are intended to be within the spirit and scope of the invention as claimed. It also is understood that the foregoing description is illustrative of the present invention and should not be considered as limiting. Therefore, other embodiments of the present invention are possible without departing from the scope of the present invention.

## Claims

1. A steerable catheter (100) comprising:
a helical coil (120) of wire (126), the helical coil (120) comprising an outer diameter; an inner diameter; a longitudinal axis (A-A); a distal end (129); a proximal end (121); and, a lumen (125) extending longitudinally from the distal end (129) to the proximal end (121), the helical coil (120) having at least a first section (120B) of loosely coiled loops (123) defining at least one gap (127);
a ribbon (140) having a distal end (149) and a proximal end (141), the ribbon (140) extending longitudinally, adjacent to the longitudinal axis (A-A), within the lumen (125), from the distal end (129) of the helical coil (120) substantially to the proximal end (121) of the helical coil (120);
a tip (130) comprising a laser-welded fusion (131) of the distal end (129) of the helical coil (120) and the distal end (149) of the ribbon (140), the tip (130) being arranged to close the distal end (129) of the helical coil (120), and having a diameter substantially equal to or less than the outer diameter of the helical coil (120), wherein the tip (130) is operatively arranged to move in a first direction away from the longitudinal axis (A-A) when the ribbon (140) is forced in a first longitudinal direction, and move in a second direction away from the longitudinal axis (A-A) when the ribbon (140) is forced in a second longitudinal direction.

2. The steerable catheter (100) as recited in Claim 1 wherein the helical coil (120) is at least partially covered in a coating (90).

3. The steerable catheter (100) as recited in Claim 1 wherein the helical coil (120) and the ribbon (140) both comprise stainless steel.

4. The steerable catheter (100) as recited in Claim 1 wherein the tip (130) is substantially spherical.

5. The steerable catheter (100) as recited in Claim 1 wherein the wire (126) has a substantially circular cross-section.

6. The steerable catheter (100) as recited in Claim 1 wherein the laser-welded fusion (131) is made with a Nd-YAG laser welder.

7. A method of making a steerable catheter (100) comprising:
providing a helical coil (120) of wire (126)of laser weldable material, the helical coil (120) comprising a longitudinal axis (A-A), a distal end (129), a proximal end (121);, and a lumen (125) extending longitudinally from the distal (129) end to the proximal end (121);
providing a ribbon (140) of laser weldable material having a distal end (149) and a proximal end, the ribbon (140) extending longitudinally, adjacent to the longitudinal axis (A-A), within the lumen (125), from the distal end (129) of the helical coil (120) past the proximal end (121) of the helical coil (120);
applying a first set of pulses from a laser welder to the distal end (149) of the ribbon (140) and the distal end (129) of the helical coil (120), the first set of pulses being sufficient to generate a molten fusion of the distal end (149) of the ribbon (140) and the distal end (129) of the helical coil (120); and,
applying a second set of pulses from a laser welder to the molten fusion sufficient to shape the molten fusion into a tip arranged to close the distal end (129) of the helical coil (120).

8. The method recited in Claim 7 wherein both the helical coil (120) and the ribbon (140) comprise stainless steel.

9. The method recited in Claim 7 wherein the helical coil (120) further comprises at least a first section (120B) of loosely coiled loops (123) defining at least one gap (127), an outer diameter, and an inner diameter, wherein the tip has a diameter substantially equal to or less than the outer diameter of the helical coil (120).

10. The method recited in Claim 7 wherein the helical coil (120) is at least partially covered in a coating (90).

11. The method recited in Claim 7 wherein the tip is operatively arranged to move in a first direction away from the longitudinal axis (A-A) when the ribbon (140) is forced in a first longitudinal direction, and move in a second direction away from the longitudinal axis (A-A) when the ribbon (140) is forced in a second longitudinal direction.

12. The method recited in Claim 7 wherein the laser welder is a pulse Nd-YAG micro laser welder.

13. The method as recited in Claim 7 wherein the tip is substantially spherical.

14. The method as recited in Claim 7 wherein the wire (126) has a substantially circular cross-section.

15. The steerable catheter (100) made according to the method recited in one of the Claims 7 to 14.
